Europäisches Patentamt

European Patent Office

Office européen des brevets

(11) Publication number: **0 333 449**
**A1**

## (12) EUROPEAN PATENT APPLICATION

(21) Application number: **89302530.4**

(22) Date of filing: **15.03.89**

(51) Int. Cl.⁴: **A 61 B 5/02**

(30) Priority: **15.03.88 JP 60720/88**

(43) Date of publication of application:
**20.09.89 Bulletin 89/38**

(84) Designated Contracting States:
**DE FR GB IT NL SE**

(71) Applicant: **COLIN ELECTRONICS CO., LTD.**
**2007-1, Hayashi**
**Komaki-shi Aichi-ken (JP)**

(72) Inventor: **Niwa, Minoru**
**c/o Colin Electronics Company Ltd. 2007-1 Hayashi**
**Komaki-shi Aichi-ken (JP)**

**Yokoe, Hifumi**
**c/o Colin Electronics Company Ltd. 2007-1 Hayashi**
**Komaki-shi Aichi-ken (JP)**

(74) Representative: **Senior, Alan Murray et al**
**J.A. KEMP & CO 14 South Square Gray's Inn**
**London WC1R 5EU (GB)**

(54) **Method and apparatus for detecting pulse wave.**

(57) A pulse wave detecting method, including the steps of pressing a sensor (20) against an artery (12) via a body surface (11), with a continuously variable pressing force, detecting amplitudes of pulses of pulse wave with the sensor while the pressing force is continuously varied, storing the amplitudes of the pulses and the continuously varied pressing force, selecting a maximum pulse ($M_{max}$) having a maximum amplitude from the stored pulses, determining as an optimum pressing force ($P_{opt}$) a pressing force at a time of detection of the maximum pulse, and pressing the sensor against the artery with the optimum pressing force. A pulse wave detecting apparatus, including a sensor (20) for detecting amplitudes of pulses of pulse wave, a pressing device (18, 22, 28, 30) for pressing the sensor against an artery via a body surface (11), a device (42) for storing the amplitudes of the pulses detected by the sensor while the sensor is pressed against the artery with the pressing force being continuously varied, and the continuously varied pressing force, a device (42, S5) for selecting a maximum pulse ($M_{max}$) having a maximum amplitude from the stored pulses and determining as an optimum pressing force ($P_{opt}$) a pressing force at a time of detection of the maximum pulse, and a device (42, S6) for commanding the pressing device to press the sensor against the artery with the optimum pressing force.

FIG. I

EP 0 333 449 A1

Description

## METHOD AND APPARATUS FOR DETECTING PULSE WAVE

The present invention generally relates to a method and an apparatus for detecting pulse waves using a pulse wave sensor pressed against an arterial vessel via a body surface of a subject.

There has been proposed a pulse wave detecting apparatus having (a) a pulse wave sensor set on a body surface of a subject under which an arterial vessel extends, for example, on a wrist surface under which a radial artery extends, and (b) a tightening band or other pressing means for pressing the pulse wave sensor against the arterial vessel via the body surface, so that the pulse wave sensor detects pulse wave, that is, oscillatory pressure wave produced from the arterial vessel synchronously with heartbeat of the subject.

In the above apparatus, the pulse wave sensor is pressed against the body surface with a pressing force which is predetermined so that pulse wave having an appropriate amplitude is detected by the sensor. The predetermined pressing force is produced by, for example, fastening the tightening band around the wrist such that a coil spring associated with the band is stretched by a predetermined length.

However, if the pulse wave sensor is used on different subjects as indicated above, namely, with the same predetermined pressing force, pulse waves detected from the subjects by the sensor would probably have different amplitudes. Furthermore, if the pulse wave sensor is set at different positions on a body surface of a subject with a predetermined pressing force, pulse waves detected at the positions by the sensor would probably have different amplitudes. Thus, the above-indicated apparatus is not capable of detecting, from every subject, an optimum pluse wave having a maximum amplitude. Thus, the accuracy of detection of pulse waves is not assured for every subject by this apparatus.

It is therefore an object of the present invention to provide a method and an apparatus wherein the pulse wave sensor is pressed against an arterial vessel with an optimum pressing force so that optimum pulse waves having a maximum amplitude are detected from the arterial vessel by the pulse wave sensor.

According to a first aspect of the present invention, there is provided a method of detecting pulse waves produced from an arterial vessel of a subject, the pulse wave consisting of a plurality of pulses, the method comprising the steps of (a) pressing a pulse wave sensor against the arterial vessel via a body surface of the subject, with a variable pressing force, (b) detecting amplitudes of pulses of the pulse wave with the pulse wave sensor, while the pressing force is varied, (c) storing the amplitudes of the pulses and the varied pressing force, (d) selecting a maximum pulse having a maximum amplitude from the stored pulses, (e) determining, as an optimum pressing force, the pressing force at a time of detection of the maximum pulse, and (f) pressing the pulse wave sensor against the arterial vessel with the optimum pressing force.

In the pulse wave detecting method arranged as described above, the pulse wave sensor is pressed against the arterial vessel while the pressing force is preferably continuously varied, so that amplitudes of pulses of pulse wave are detected with the pressing force being continuously varied, and that both the amplitudes of the pulses and the continuously varied pressing force are stored. Further, a maximum pulse having a maximum amplitude is selected from the stored pulses, a pressing force at the time of detection of the maximum pulse is determined as an optimum pressing force, and the pulse wave sensor is then pressed against the arterial vessel with the optimum pressing force.

Thus, in the present method, an optimum pressing force is determined on each subject, and the pulse wave sensor is pressed with the optimum pressing force so as to detect pulse wave having a maximum amplitude from each subject. Accordingly, the present method assures improved accuracy of detection of pulse wave, whereby accurate blood pressure is determined based on the pulse wave and clear and accurate waveform of the pulse wave is obtained.

According to a second aspect of the present invention, there is provided an apparatus for detecting pulse wave produced from an arterial vessel of a subject, the pulse wave consisting of a plurality of pulses, the apparatus comprising (1) a pulse wave sensor for detecting amplitudes of pulses of the pulse wave, (2) pressing means for pressing the pulse wave sensor against the arterial vessel via a body surface of the subject, with a pressing force, (3) storing means for storing the amplitudes of the pulses of the pulse wave detected by the pulse wave sensor while the pulse wave sensor is pressed against the arterial vessel with the pressing force of the pressing means being varied, and the varied pressing force of the pressing means, (4) determining means for selecting a maximum pulse having a maximum amplitude from the stored pulses, and determining, as an optimum pressing force, a pressing force of the pressing means at a time of detection of the maximum pulse, and (5) commanding means for commanding the pressing means to press the pulse wave sensor against the arterial vessel with the optimum pressing force.

The pulse wave detecting apparatus constructed as described above has the same advantages as those of the method according to the first aspect of the invention.

In a preferred embodiment of the apparatus of the invention, the pressing means presses the pulse wave sensor against the arterial vessel with a pressing force which is continuously varied between a first level and a second level higher than the first level, the storing means storing the amplitudes of the pulses of the pulse wave detected by the pulse wave sensor while the pressing force is continuously varied between the first level and the second level.

According to a preferred feature of the above embodiment of the invention, the pressing means presses the pulse wave sensor against the arterial vessel with a pressing force which is increased to the first level at a first increase rate and increased from the first level to the second level at a second increase rate lower than the first increase rate.

According to another preferred feature of the same embodiment of the invention, the first level is not less than zero mmHg, preferably the first level is 20 mmHg, and the second level is 80 mmHg, and preferably the difference between the first and second levels is not less than 50 mmHg.

According to another preferred feature of the same embodiment of the invention, the second increase rate falls in a range of 5 to 6 mmHg per second.

In another embodiment of the apparatus of the invention, the pressing means includes a housing in which the pulse wave sensor is accommodated, a diaphragm disposed between the housing and the pulse wave sensor such that the pulse wave sensor is supported by the housing via the diaphragm, the diaphragm cooperates with at least the housing to define a fluid-tight space in the housing, an electric pump for supplying pressurized fluid to the fluid-tight space, and a presure regulating valve for regulating pressure in the fluid-tight space so as to continuously increase the pressing force with which the pulse wave sensor supported by the diaphragm is pressed against the arterial vessel, the storing means storing the amplitudes of the pulses of the pulse wave detected by the pulse wave sensor while the pressure in the fluid-tight space is continuously increased, the determining means selecting the maximum pulse from the stored pulses and determining, as an optimum pressure, a pressure in the fluid-tight space at a time of detection of the maximum pulse, the optimum pressure serving as the optimum pressing force, the commanding means commanding the pressure regulating valve to maintain the pressure in the fluid-tight space, at the optimum pressure. In this embodiment, the second level may be not more than a maximum permissible pressure which is produced in the fluid-tight space by the electric pump.

In yet another embodiment according to the second aspect of the invention, the apparatus further comprises means for determining blood pressure of the subject based on the pulse wave detected by the pulse wave sensor while the pressing force of the pressing means is maintained at the optimum pressing force, and means for displaying the determined blood pressure.

In a further embodiment according to the second aspect of the invention, the apparatus further comprises means for displaying waveform of the pulse wave detected by the pulse wave sensor while the pressing force of the pressing means is maintained at the optimum pressing force.

BRIEF DESCRIPTION OF THE DRAWINGS

The above and optional objects, features and advantages of the present invention will be better understood by reading the following detailed description of the presently preferred embodiment of the invention, when considered in conjunction with the accompanying drawings, in which:

Fig. 1 is an illustrative view of a pulse wave detecting apparatus of the present invention;

Fig. 2 is a flow chart illustrating the operation of the apparatus of Fig. 1; and

Figs. 3(a) and 3(b) are graphs respectively showing time-wise varying pressure P in a housing of a pulse wave detecting probe of the apparatus of Fig. 1, and time-wise varying magnitude (i.e., wave form) of pulse wave detected by a pulse wave sensor of the pulse wave detecting probe, in comparison with each other. DETAILED DESCRIPTION OF THE PREFERRED EMBODIMENT

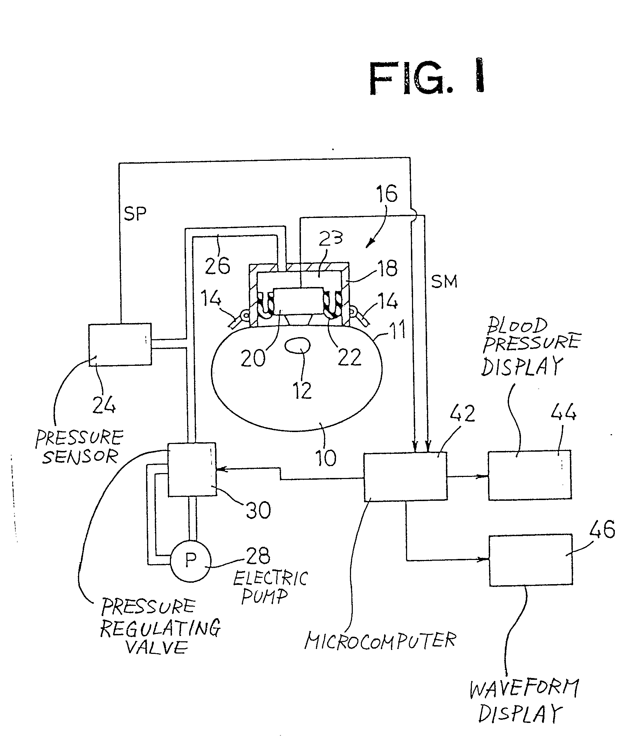

Referring first to Fig. 1, there is illustrated a pulse wave detecting apparatus embodying the present invention. In the figure, reference numeral 10 designates a body portion of a subject such as a wrist. An arterial vessel 12 such as a radial artery extends near a surface (skin) 11 of the wrist 10. A pulse wave detecting probe 16 is fixed on the wrist surface 11 with a fastening band 14, such that a pulse wave sensor 20 of the probe 16 is placed right above the radial artery 12 via the wrist surface 11.

In addition to the pulse wave sensor 20, the pulse wave detecting probe 16 includes a box-like housing 18 with a comparatively high rigidity, and a generally annular elastic diaphragm 22 formed of rubber. The housing 18 has an opening in its bottom as viewed in Fig.1. The pulse wave sensor 20 includes a pressure-sensitive semiconductor element, and is accommodated in the housing 18. The diaphragm 22 is disposed between the housing 18 and the sensor 20, such that the sensor 20 is supported by the housing 18 via the diaphragm 22, and that the diaphragm 22 cooperates with the housing 18 and the sensor 20 to define a fluid-tight space 23 in the housing 18. The fluid-tight space 23 communicates with an electrically operated pump 28 and a pressure-regulating valve 30 via a piping 26. The pressure regulating valve 30 is controlled by a microcomputer 42 (described below) so as to regulate fluid supply from the electric pump 28 to the fluid-tight space 23 and thereby vary and maintain pressure P in the space 23. Upon the supply of pressurized fluid from the electric pump 28 to the fluid-tight space 23, pressure P in the space 23 is increased and the elastic diaphragm 22 is expanded toward the wrist surface 11, whereby the pulse wave sensor 20 is pressed on the wrist surface 11 with a pressing force.

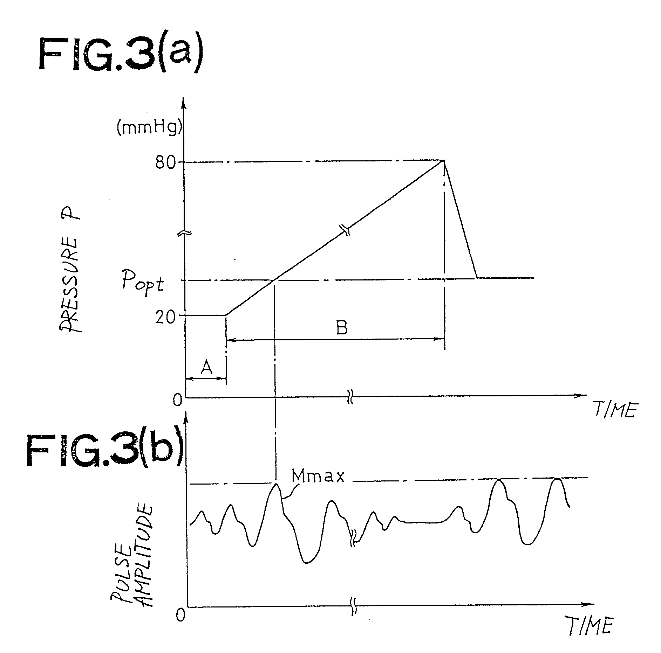

While being pressed on the wrist surface 11, the pulse wave sensor 20 detects pulse wave, that is, oscillatory pressure wave transmitted thereto from the wrist 10 due to expansion and contraction of the radial artery 12 which are synchronous with heartbeat of the subject. The sensor 20 generates pulse wave signal SM representing the detected pulse wave, to the microcomputer 42. The pulse wave consists of pulses corresponding to heartbeats (see Fig. 3(b)). In the present embodiment, the housing 18, diaphragm 22, pump 28 and valve 30 cooperate with each other to serve as the means for pressing the pulse wave sensor against the arterial vessel via the body surface, with a pressing force.

A pressure sensor 24 is connected to the piping 26 at a location between the housing 18 and the pressure regulating valve 30, and generates to the microcomputer 42 pressure signal SP representing pressure P in the piping 26, namely, in the fluid-tight space 23 of the housing 18.

The microcomputer 42 includes a central processing unit (CPU), a random access memory (RAM), a read only memory (ROM) and an I/0 (input/output) interface (all not shown). The CPU processes the received signals according to software programs pre-stored in the ROM by utilizing temporary-storage function of the RAM, so as to control the pressure-regulating valve 30 and thereby regulate pressure P in the housing space 23. More specifically described, upon operation of a start switch (not shown), the microcomputer 42 activates the pressure regulating valve 30 so as to permit fluid supply from the electric pump 28 to the housing space 23 and thereby continuously increase pressure P in the space 23. When pressure P is continuously increased, the pulse wave senscr 20 consecutively detects amplitudes of pulses of pulse wave and generates signal SM representing the detected amplitudes to the microcomputer 42. Concurrently the pressure sensor 24 detects the continuously decreased pressure and generates signal SP representing the detected continuously increased pressure to the microcomputer 42. The microcomputer 42 time-wise stores in the RAM the amplitudes of the pulses of the pulse wave represented by signal SM, in association with the continuously increased pressure of the housing space 23 represented by signal SP. The microcomputer 42 selects a maximum pulse $M_{max}$ having a maximum amplitude from all the stored pulses, and determines as an optimum pressure $P_{opt}$ a pressure at the time of detection of maximum pulse $M_{max}$. Further, the microcomputer 42 controls the pressure regulating valve 30 so as to maintain pressure P at optimum value $P_{opt}$. In the present embodiment, the RAM of the microcomputer 42 serves as the means for storing the amplitudes of the pulses of the pulse wave detected by the pulse wave sensor while the pressing force of the pressing means is continuously varied, and the continuously varied pressing force of the pressing means.

Experiences teaches that optimum pressure $P_{opt}$ generally falls in the range of about 20 mmHg to about 80 mmHg throughout all subjects. Accordingly, pressure P is increased at a comparatively high rate from zero mmHg to the lower limit, 20 mmHg, and subsequently from the lower limit to the upper limit, 80 mmHg, at a compartively low rate of 5 to 6 mmHg/sec which rate is suitable to detect a sufficient number of pulses of pulse wave. Thus, it needs a comparatively short time of about ten seconds to increase pressure P to the upper limit, and therefore it requires a shortened time to determine optimum pressure $P_{opt}$. Thus, this ar-

rangement leads to minimizing uncomfortable feeling of the subject caused by pressing the pulse wave sensor 20 against the wrist surface 11 with the continuously variable pressing force.

The microcomuter 42 determines blood pressure of the subject based on each of pulses of pulse wave detected by the pulse wave sensor 20 while pressure P in the housing space 23 is maintained at optimum value $P_{opt}$, and commands a blood pressure display 44 to display the determined blood pressure and commands a waveform display 46 to display the waveform of the pulses of the pulse wave. More specifically described, maximum blood pressure corresponds to a magnitude of the upper peak of each of pulses of pulse wave while minimum blood pressure corresponds to a magnitude of the lower peak of the same pulse, and therefore maximum and minimum blood pressure is determined according to a predetermined relationship between pulse wave magnitude and blood pressure based on actually detected magnitudes of the upper and lower peaks of each pulse of pulse wave. The pulse wave displayed on the waveform display 46 consists of maximum pulses having maximum amplitudes, and therefore those pulses have clear and good waveforms which are clinically or medically very informative.

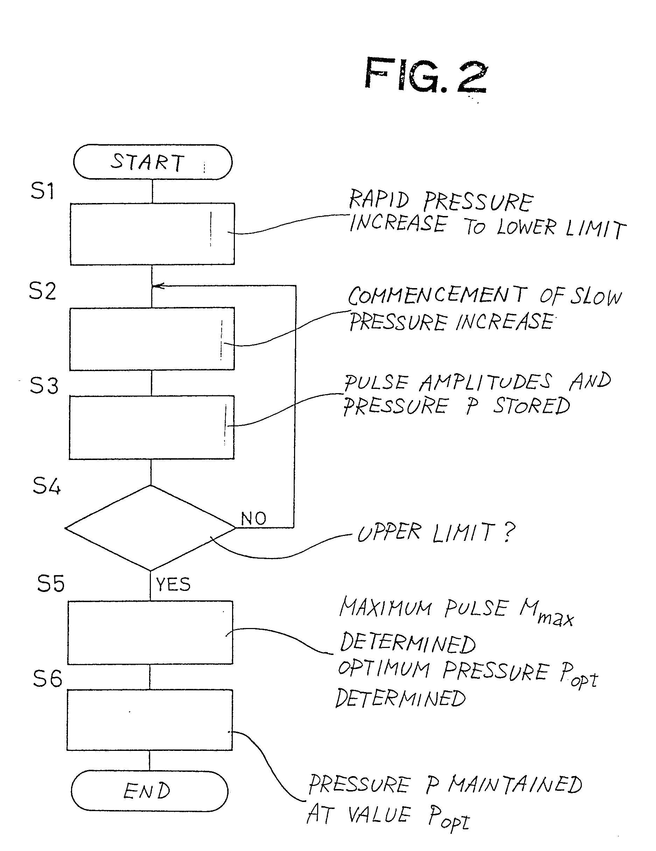

Referring next to Fig. 2, there is illustrated a flow chart according to which the pulse wave detecting apparatus constructed as described above is operated to regulate pressure P in the fluid-tight space 23 of the housing 18 and thereby control a pressing force with which the pulse wave sensor 20 is pressed against the radial artery 12 via the wrist surface 11.

Upon application of electric power to the present apparatus as a result of operation of the start switch (not shown), the control of the CPU of the microcomputer 42 goes to step S1 at which the electric pump 28 is activated so that pressure P in the housing space 18 is increased at a comparatively high rate from zero mmHg to the lower limit, 20 mmHg. Subsequently pressure P is maintained at the lower limit for about two seconds as indicated at A in the graph of Fig. 3(a). A contact surface of the pulse wave sensor 20 may not be in close contact with the wrist surface 11 immediately after the rapid pressure increase, and if so noise may be mixed with pulse wave signal SM. Thus, the waiting time A is provided to prevent noise from being mixed with signal SM.

Step S1 is followed by step S2 at which pressure P is increased at a comparatively low rate of 5 to 6 mmH/sec to the upper limit, 80 mmHg, as indicated at B in the graph of Fig. 3(a). At the following step S3 the amplitudes of pulses of pulse wave and the continuously increased pressure which are detected by the pulse wave sensor 20 and the pressure sensor 24, respectively, in the time period B, are time-wise stored in the RAM.

Step S3 is followed by step S4 at which it is judged whether or not pressure P has reached the upper limit (80 mmHg). If the judgement at step S4 is negative (NO), steps S2 and S3 are repeated until the judgement at step S4 is turned affirmative (YES).

Meanwhile, if the judgement at step S4 is affirmative, the control of the CPU goes to step S5 at which maximum pulse $M_{max}$ having a maximum amplitude is selected from all the pulses stored in the RAM, and a pressure in the housing space 23 at the time of detection of maximum pulse $M_{max}$ is determined as optimum pressure $P_{opt}$. Step S5 is followed by step S6 at which the pressure regulating valve 30 is controlled by the microcomputer 42 so that pressure P is decreased to optimum pressure $P_{opt}$ and maintained at optimum value $P_{opt}$ as shown in the graph of Fig. 3(a). Then, while the pulse wave sensor 20 is pressed against the radial artery 12 via the wrist surface 11 with pressure P maintained at optimum value $P_{opt}$, pulse wave is detected by the pulse wave sensor 20 and maximum and minimum blood pressure is determined based on the thus detected pulse wave. The deetermined blood pressure is displayed on the blood pressure display 44, and the waveform of the detected pulse wave is displayed on the waveform display 46. In the present embodiment, step S5 stored in the form of software program in the ROM and the microcomputer 42 for effecting step S5, serve as the means for selecting a maximum pulse having a maximum amplitude and determining an optimum pressing force based on the determined maximum pulse. Meanwhile, step S6 stored in the ROM and the microcomputer 42 for effecting step S6, serve as the means for commanding the pressing means to press the pulse wave sensor against the arterial vessel with the optimum pressing force.

As is apparent from the foregoing, in the present pulse wave detecting apparatus, a maximum pulse $M_{max}$ having a maximum amplitude is selected from the pulses detected by the pulse wave sensor 20 while pressure P is continuously increased, and a pressure at the time of detection of maximum pulse $M_{max}$ is determined as optimum pressure $P_{opt}$. With pressure P maintained at optimum value $P_{opt}$, the pulse wave sensor 20 is pressed against the radial artery 12 with an optimum pressing force. Different optimum pressing forces are determined on different subjects, so that a maximum pulse $M_{max}$ is detected from each of the different subjects. Thus, the present apparatus is capable of detecting pulse wave with an extremely improved accuracy, in contrast to the conventional apparatus in which a pulse wave sensor thereof is pressed against a body surface with a predetermined pressing force irrespective of differences among subjects.

While the present invention has been described in its presently preferred embodiment with detailed particularities, the invention may be embodied with various modifications.

For example, while in the illustrated embodiment pressure P in the housing space 23 is increased at the comparatively high rate to the lower limit, 20 mmHg, and subsequently at the comparatively low rate of 5 to 6 mmHg/sec to the upper limit, 80 mmHg, so as to shorten an overall time required to detemine optimum pressure $P_{opt}$ at which maximum pulse $M_{max}$ is detected, it is possible to increase pressure P at the comparatively low rate of 5 to 6 mmHg/sec from the lower limit of zero mmHg throughout to the upper limit of about 80 mmHg and determine optimum pressure $P_{opt}$ in the course of the slow pressure increase.

Although in the illustrated embodiment the waiting time A of about two seconds is provided between the rapid and slow pressure increases, it is possible to omit the waiting time A.

While in the illustrated embodiment the lower limit to which pressure P is increased at the comparatively high rate and the upper limit to which pressure P is increased at the comparatively low rate, are predetermined at 20 mmHg and 80 mmHg, respectively, it is possible to employ as the lower limit a pressure not less than zero mmH and as the upper limit a pressure not more than a maximum permissible pressure which is produced in the housing space 23 by the electric pump 28. Furthermore, it is preferred that a difference between the upper and lower limits be not less than about 50 mmHg.

In a modified form of the illustrated embodimemt, there is provided an aberrant signal detecting routine between steps S3 and S4 of the flow chart of Fig. 2. In this routine, it is judged whether or not an aberrant signal has been detected and stored at step S3. For example, when the subject moves the wrist 10 around which the pulse wave detecting probe 16 is wound, an aberrant signal may be mixed with pulse wave singal SM and stored in the RAM. If the judgement is affirmative, the slow pressure increase is ceased, pressure P is decreased to a pressure at the time of detection of the aberrant signal, and subsequently pressure P is re-increased from that pressure level at the comparatively low rate so as to detect pulses of pulse wave. Alternatively, in such case it is possible that pressure P is decreased to the lower limit and re-increased from the lower limit.

In another modified form of the illustrated embodiment, the microcomputer 42 is adapted to subject pulse wave signal SM supplied from the pulse wave sensor 20, to "median filter" in which each of pulses of pulse wave represented by signal SM, is replaced with a median pulse which has a median amplitude in three pulses consisting of the each pulse and a pair of pulses preceding and following the each pulse. In this case, noise mixed with pulse wave singal SM due to physical motion of the subject, for example, is preferably removed, and accordingly the selection of maximum pulse $M_{max}$ having a maximum amplitude is effected with higher reliability.

It is to be understood that the present invention may be embodied with other modifications, changes and improvements that may occur to those skilled in the art without departing from the scope and spirit of the invention defined in the appended claims.

## Claims

1. A method of detecting pulse wave produced from an arterial vessel (121) of a subject, said pulse wave consisting of a plurality of pulses, the method comprising the steps of: pressing a pulse wave sensor (20) against said arterial vessel via a body surface (11) of the subject, with a variable pressing force,
detecting amplitudes of pulses of said pulse wave with said pulse wave sensor, while the pressing force is varied,
storing said amplitudes of the pulses and the varied pressing force,
selecting a maximum pulse ($M_{max}$) having a maximum amplitude from the stored pulses,
determining, as an optimum pressing force ($P_{opt}$),the pressing force at the time of detection of said maximum pulse, and then
pressing said pulse wave sensor against said arterial vessel with said optimum pressing force.

2. An apparatus for detecting pulse wave produced from an arterial vessel (12) of a subject, said pulse wave consisting of a plurality of pulses, the apparatus comprising:
a pulse wave sensor (20) for detecting amplitudes of pulses of said pulse wave;
pressing means (18, 22, 28, 30) for pressing said pulse wave sensor against said arterial vessel via a body surface (11) of the subject, with a pressing force;
storing means (42) for storing the amplitudes of the pulses of said pulse wave detected by said pulse wave sensor while said pulse wave sensor is pressed against said arterial vessel with said pressing force of said pressing means being varied, and the varied pressing force of said pressing means;
determining means (42, S5) for selecting a maximum pulse ($M_{max}$) having a maximum amplitude from the stored pulses, and determining, as an optimum pressing force ($P_{opt}$),the pressing force of said pressing means at the time of detection of said maximum pulse; and
commanding means (42, S6) for commanding said pressing means to press said pulse wave sensor against said arterial vessel with said optimum pressing force.

3. The apparatus as set forth in claim 2, wherein said pressing means (18, 22, 28, 30) presses said pulse wave sensor (20) against said arterial vessel (12) with a pressing force which is continuously varied between a first level and a second level higher than said first level, said storing means (42) storing the amplitudes of the pulses of said pulse wave detected by said pulse wave sensor while said pressing force is continuously varied between said first level and said second level.

4. The apparatus as set forth in claim 3, wherein said pressing means (18, 22, 28, 30) presses said pulse wave sensor (20) against said arterial vessel (12) with a pressing force which is increased to said first level at a first increase rate and increased from said first level to said second level at a second increase rate lower than said first increase rate.

5. The apparatus as set forth in claim 3, said first level is not less than zero mmHg.

6. The apparatus as set forth in claim 3 or claim 4, wherein said first level is 20 mmHg, and said second level is 80 mmHg.

7. The apparatus as set forth in claim 3, 4 or 5, wherein a difference between said first and second levels is not less than 50 mmHg.

8. The apparatus as set forth in claim 4, 5, 6 or 7 wherein said second increase rate falls in the range of 5 to 6 mmHg per second.

9. The apparatus as set forth in any one of claims 2 to 8, wherein said pressing means includes
a housing (18) in which said pulse wave sensor (20) is accommodated,
a diaphragm (22) disposed between said housing and said pulse wave sensor such that said pulse wave sensor is supported by said housing via said diaphragm, and that said diaphragm cooperates with at least said housing to define a fluid-tight space (23) in the housing,
an electric pump (28) for supplying pressurized fluid to said fluid-tight space, and
a presure regulating valve (30) for regulating pressure in said fluid-tight space so as to continuously increase said pressing force with which the pulse wave sensor supported by said diaphragm is pressed against said arterial vessel (12),
said storing means (42) storing the amplitudes of the pulses of the pulse wave detected by said pulse wave sensor while said pressure in said fluid-tight space is continuously increased,
said determining means (42, S5) selecting said maximum pulse ($M_{max}$) from the stored pulses and determining, as an optimum pressure ($P_{opt}$), the pressure in said fluid-tight space at the time of detection of said maximum pulse, said optimum pressure serving as said optimum pressing force,
said commanding means (42, 56) commanding said pressure regulating valve to maintain said pressure in said fluid-tight space, at said optimum pressure.

10. The apparatus as set forth in claim 9, wherein said second level is not more than a maximum permissible pressure which is produced in said fluid-tight space (23) by said electric pump (28).

11. The apparatus as set forth in any one of claims 2 to 10, further comprising
means (42) for determining blood pressure of the subject based on the pulse wave detected by said pulse wave sensor (20) while the pressing force of said pressing means (18, 22, 28, 30) is maintained at said optimum pressing force ($P_{opt}$), and
means (44) for displaying the determined blood pressure. to 11, further comprising
means (46) for displaying waveform of the pulse wave detected by said pulse wave sensor (20) while the pressing force of said pressing means (18, 22, 28, 30) is maintained at said optimum pressing force ($P_{opt}$).

# FIG. 1

SP

16

23

26

18

14          14

20          22          11

24

12

10

42

PRESSURE
SENSOR

30

BLOOD
PRESSURE
DISPLAY

44

SM

P          28
ELECTRIC
PUMP

46

PRESSURE
REGULATING
VALVE

MICROCOMPUTER

WAVEFORM
DISPLAY

# FIG. 2

START

S1 — RAPID PRESSURE INCREASE TO LOWER LIMIT

S2 — COMMENCEMENT OF SLOW PRESSURE INCREASE

S3 — PULSE AMPLITUDES AND PRESSURE P STORED

S4 — NO — UPPER LIMIT?

S5 — YES — MAXIMUM PULSE $M_{max}$ DETERMINED OPTIMUM PRESSURE $P_{opt}$ DETERMINED

S6 — PRESSURE P MAINTAINED AT VALUE $P_{opt}$

END

# FIG.3(a)

# FIG.3(b)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 078 090 (NEDERLANDSE CENTRALE ORGANISATIE VOOR TOEGEPAST-NATUURWETENSCHAPPELIJK ONDERZOEK) * Abstract; page 2, line 27 - page 3, line 5; page 3, line 22 - page 5, line 15; figure 1 * | 1-7 | A 61 B 5/02 |
| A | FR-A-2 513 507 (S.A. BERSAC et al.) * Page 1, lines 1-5; page 6, lines 17-24; page 6, line 36 - page 7, line 5; page 7, lines 34-37; page 10, line 11 - page 11, line 34; figures 1,5-7 * | 1,3,11, 12 | |
| A | DE-A-3 341 597 (K. ECKEL) * Whole document * | 1-3,11, 12 | |
| A | US-A-4 423 738 (P.M. NEWGARD) * Abstract; column 2, lines 55-67; column 5, line 33 - column 6, line 21; figures 1,5,6 * | 2,9,11, 12 | |

TECHNICAL FIELDS SEARCHED (Int. Cl.4)

A 61 B

The present search report has been drawn up for all claims

| Place of search | Date of completion of the search | Examiner |
|---|---|---|
| THE HAGUE | 02-06-1989 | FERRIGNO, A. |

EPO FORM 1503 03.82 (P0401)